# FASCICULE DE BREVET EUROPEEN

(11) **EP 4 263 498 B1**
(45) Date de publication et mention de la délivrance du brevet: **22.07.2026**
(21) Numéro de dépôt: 21851835.5
(22) Date de dépôt: 16.12.2021
(51) Int. Cl.: C07C 319/08, C07C 319/28, C07C 321/04

(54) **PROCEDE DE SECHAGE DU METHYLMERCAPTAN PAR DISTILLATION AZEOTROPIQUE**
VERFAHREN ZUR TROCKNUNG VON METHYLMERCAPTAN DURCH AZEOTROPE DESTILLATION
METHOD FOR DRYING METHYL MERCAPTAN BY MEANS OF AZEOTROPIC DISTILLATION

(30) Priorité: 17.12.2020 FR 2013436
(43) Date de publication de la demande: 25.10.2023
(73) Titulaire: ARKEMA FRANCE, 92800 Puteaux (FR)
(72) Inventeur: FREMY, Georges, 64170 Lacq (FR); RAYMOND, Jean-Michel, 40300 Cauneille (FR)
(74) Mandataire: Arkema Patent
(86) Numéro de dépôt international: PCT/FR2021/052364
(87) Numéro de publication internationale: WO 2022/129801

(56) Documents cités:
- US-A- 5 866 721

## Description

La présente invention concerne un procédé de séchage du méthylmercaptan, notamment par distillation azéotropique. La présente invention concerne également des procédés de production de méthylmercaptan comprenant ledit procédé de séchage.

Les mercaptans présentent un grand intérêt industriel et sont aujourd'hui très largement utilisés par les industries chimiques, notamment comme matières premières pour la synthèse de molécules organiques plus complexes. Par exemple, le méthylmercaptan (CH₃SH ou MeSH) est utilisé comme matière première dans la synthèse de la méthionine, acide aminé essentiel pour l'alimentation animale. Le méthylmercaptan est également utilisé dans la synthèse de disulfures de dialkyles, en particulier dans la synthèse du disulfure de diméthyle (DMDS), additif de sulfuration de catalyseurs d'hydrotraitement de coupes pétrolières, entre autres applications.

La synthèse industrielle du méthylmercaptan se fait généralement selon deux voies connues. La première, appelée voie méthanol, produit le méthylmercaptan à partir de méthanol et de d'hydrogène sulfuré selon la réaction (1) suivante :

CH₃OH + H₂S - > CH₃SH + H₂O (1)

Avec ce procédé, une réaction secondaire donne lieu à la formation de diméthylsulfure, selon la réaction (2) suivante :

CH₃OH + CH₃SH -> CH₃SCH₃ + H₂O (2)

La seconde voie, appelée voie oxyde de carbone, permet d'obtenir le méthylmercaptan à partir d'un oxyde de carbone, d'hydrogène, d'hydrogène sulfuré et/ou de soufre, par exemple selon les réactions (3) et (4) suivantes :

CO + 2H₂ + H₂S -> CH₃SH + H₂O (3)

CO + S + 3H₂ -> CH₃SH + H₂O (4)

Comme l'indiquent les réactions précédentes, la synthèse de méthylmercaptan s'accompagne d'une production d'eau quelle que soit la voie utilisée. Ainsi, il est ensuite nécessaire de séparer le méthylmercaptan de l'eau. Cependant, l'eau est légèrement soluble dans le méthylmercaptan. Il en reste donc toujours dans le produit obtenu, à éliminer autant que possible.

En effet, il existe des applications industrielles pour lesquelles une teneur très basse en eau résiduelle dans le méthylmercaptan est souhaitable. Par exemple, dans la synthèse du diméthyldisulfure par oxydation au soufre du méthylmercaptan, l'eau peut rendre moins actif les catalyseurs de cette réaction.

De plus, lorsque la teneur en eau résiduelle dans le méthylmercaptan est élevée (notamment de l'ordre de quelques milliers de ppm), et que la température est en dessous de 16°C environ, une partie de l'eau peut se désolubiliser, décanter et favoriser la formation d'hydrates de méthylmercaptan solides. Ces résidus solides peuvent conduire à des risques de bouchage des équipements, entrainant des problèmes majeurs de sécurité des installations et de transport.

Pour éviter ces risques, le séchage du méthylmercaptan est classiquement effectué par adsorption de l'eau sur des tamis moléculaires. Cependant, cette méthode présente de nombreux inconvénients.

Par exemple, la régénération des tamis moléculaires se fait à des températures élevées et conduit à la formation de sous-produits indésirables tels que le diméthylsulfure.

De plus, lorsque le méthylmercaptan est formé par voie méthanol, des traces de méthanol dans le méthylmercaptan à sécher peuvent drastiquement faire chuter la capacité d'adsorption en eau des tamis moléculaires. Ceci implique d'augmenter la fréquence des régénérations, ce qui augmente les coûts de production et la formation de sous-produits indésirables. Le document US 5,866.721 décrit un procédé de purification de méthylmercaptan. Plus particulièrement, le méthylmercaptan est séparé du diméthytsulfure (DMS) par une distillation. A l'issue de cette distillation, le méthylmercaptan et l'eau sont récupérés en tète de colonne tandis que le DMS est récupéré en pied de colonne. Le distillât récupéré en tète de colonne est condensé, puis séparé en méthylmercaptan et en eau.

Il existe donc un besoin pour un méthylmercaptan avec une faible teneur en eau, de préférence avec une teneur en eau la plus faible possible.

Il existe également un besoin pour un procédé de séchage du méthylmercaptan qui soit efficace et qui permette d'éviter en tout ou partie les désavantages des procédés de séchage connus.

Un objectif de la présente invention est de proposer un procédé de séchage du méthylmercaptan, qui permette d'obtenir un méthylmercaptan avec une faible teneur en eau, de préférence avec une teneur en eau inférieure ou égale à 1500 ppm.

Un autre objectif de la présente invention est de fournir un procédé permettant de pallier en tout ou partie les inconvénients des procédés de séchage jusqu'alors utilisés et notamment utilisant des tamis moléculaires.

Un objectif de la présente invention est également de fournir un procédé dont les modalités de séchage sont contrôlées et/ou ne varient pas au cours du temps.

Un objectif de la présente invention est de fournir un procédé de préparation de méthylmercaptan qui permette d'obtenir un méthylmercaptan à faible teneur en eau, de préférence avec une teneur en eau inférieure ou égale à 1500 ppm.

Un objectif de la présente invention est de fournir un procédé de préparation de méthylmercaptan intégré, qui soit plus respectueux de l'environnement et plus économique.

La présente invention répond en tout ou partie aux objectifs ci-dessus.

Le méthylmercaptan et l'eau peuvent former un mélange azéotrope, de préférence un hétéroazéotrope. On entend notamment par « mélange azéotrope » un mélange liquide qui bout en gardant une composition fixe (la phase vapeur a la même composition que la phase liquide). De préférence, le méthylmercaptan et l'eau forment un mélange azéotrope à une pression comprise entre 0,05 et 75 bars absolus, de préférence entre 1 et 30 bars absolus, plus préférentiellement entre 5 et 15 bars absolus.

Ainsi les présents inventeurs ont découvert qu'une distillation, de préférence azéotropique, permet de procéder au séchage du méthylmercaptan.

De façon surprenante, le procédé selon l'invention permet en effet de sécher efficacement le méthylmercaptan. En particulier, le procédé de séchage selon l'invention permet d'obtenir un méthylmercaptan comprenant entre 0 et 1500 ppm d'eau.

Contrairement aux tamis moléculaires, le procédé de séchage selon l'invention permet également de conserver des modalités de séchage qui ne sont pas altérées au cours du temps et qui sont facilement contrôlables en fonction des conditions opératoires, notamment des conditions de température et de pression. En particulier, il est possible de contrôler et/ou de choisir la teneur en eau du méthylmercaptan obtenu grâce au procédé de séchage selon l'invention.

De plus, ledit procédé de séchage évite les cycles de régénération des tamis moléculaires et évite ainsi la formation additionnelle de diméthylsulfure (DMS), sous-produit non désiré (parfois incinéré comme déchet).

Le procédé de séchage selon l'invention est facile à mettre en œuvre et peut être adapté à toute installation de production de méthylmercaptan, notamment par voie méthanol ou par voie oxyde de carbone. On peut ainsi obtenir un procédé de production de méthylmercaptan intégré, plus respectueux de l'environnement et plus économique.

Selon la présente invention, l'unité ppm (partie par million) fait référence à une fraction massique.

Selon la présente invention, l'expression « compris entre X et X » inclut les bornes décrites.

Par « séchage », on entend l'élimination de l'eau.

On entend notamment par « méthylmercaptan séché », le méthylmercaptan issu du procédé de séchage selon l'invention. Le flux (F) défini ci-après peut comprendre, voire être constitué dudit méthylmercaptan séché.

En particulier, on entend par « méthylmercaptan séché » un méthylmercaptan comprenant entre 0 et 1500 ppm, de préférence entre 0 et 1000 ppm, par exemple entre 10 et 800 ppm, plus préférentiellement entre 40 et 800 ppm d'eau, par rapport au total en poids du méthylmercaptan et de l'eau.

On peut également entendre par « méthylmercaptan séché » une composition comprenant du méthylmercaptan et entre 0 et 1500 ppm, de préférence entre 0 et 1000 ppm, par exemple entre 10 et 800 ppm, plus préférentiellement entre 40 et 800 ppm d'eau, par rapport au total en poids du méthylmercaptan et de l'eau.

Eventuellement, ledit méthylmercaptan séché peut également comprendre des traces de méthanol, d'H₂S et de sous-produits soufrés. On entend par « traces » d'un composé, une quantité comprise entre 0 et 1000 ppm. En particulier, les sous-produits soufrés sont le diméthylsulfure et le diméthyldisulfure.

Le méthylmercaptan séché peut être à l'état liquide ou gazeux, de préférence à l'état liquide.

On entend notamment par « étape de purification du méthylmercaptan », une étape permettant d'obtenir un flux enrichi en méthylmercaptan. On entend notamment par « flux enrichi en méthylmercaptan » un flux qui comprend un pourcentage en poids de méthylmercaptan (par rapport au poids total dudit flux) supérieur au pourcentage en poids de méthylmercaptan par rapport au poids total dudit flux avant ladite étape de purification.

### Procédé de séchage du méthylmercaptan

La présente invention concerne un procédé de séchage de méthylmercaptan comprenant les étapes suivantes :
1) on introduit un flux (**A**) comprenant du méthylmercaptan et de l'eau dans une colonne de distillation (**1**) ;
2) on distille ledit flux (**A**) dans ladite colonne (**1**) ;
3) on récupère le distillat (**B**) à l'état gazeux, de préférence en tête de colonne ;
4) on condense le distillat (**B**), de préférence dans un condenseur (**2**), de façon à obtenir un condensat (**C**) à l'état liquide ;
5) on sépare, de préférence à l'aide d'un décanteur (**3**), ledit condensat (**C**) de façon à obtenir deux phases liquides séparées :
   - une phase aqueuse (**D**) ; et
   - une phase organique (**E**) comprenant du méthylmercaptan ;
6) éventuellement on introduit en tout ou partie la phase organique (**E**) dans la colonne de distillation (**1**) en tant que reflux ; et
7) on récupère un flux (**F**) comprenant le méthylmercaptan séché en fond de la colonne (**1**).

Le flux (F) correspond notamment au méthylmercaptan séché tel que défini ci-dessus. Il est récupéré de la colonne de distillation en fond de colonne de distillation.

La distillation de l'étape 2) peut être effectuée à une pression comprise entre 0,05 et 75 bars absolus, de préférence entre 1 et 30 bars absolus, plus préférentiellement entre 5 et 15 bars absolus, par exemple à environ 10, 11, 12, 13, 14 ou 15 bars absolus.

La distillation de l'étape 2) peut être effectuée à une température comprise entre 20°C et 200°C, de préférence entre 60°C et 100°C, plus préférentiellement entre 65°C et 95°C ; par exemple entre 70°C et 90°C. De façon préférée à la distillation de l'étape 2) peut être effectuée à une température comprise entre 40 °C et 200 °C, de préférence entre 80°C et 100°C en fond de colonne, et entre 20° C et 100°C, de préférence entre 60°C et 80°C en tête de colonne.

De façon particulièrement préférée, la distillation de l'étape 2) est effectuée à une pression comprise entre 5 et 15 bars absolus et à une température comprise entre 60°C et 100°C. En particulier, la distillation de l'étape 2) est effectuée à une pression comprise entre 5 et 15 bars absolus et à une température comprise entre 70°C et 90°C. En particulier, la distillation de l'étape 2)est une distillation azéotropique.

La distillation de l'étape 2) peut être réalisée dans n'importe quel type de colonne à distiller connue. Il peut s'agir d'une colonne à plateaux (par exemple plateaux à calottes, plateaux à soupapes ou plateaux perforés) ou à garnissage (par exemple à garnissage en vrac ou structuré). La distillation de l'étape 2) peut être réalisée dans une colonne à plateaux, de préférence comprenant entre 5 et 50 plateaux, plus préférentiellement entre 10 et 40 plateaux, par exemple entre 25 et 30 plateaux. La distillation de l'étape 2) peut également être effectuée dans une colonne à cloison (appelée DWC en langue anglaise pour Divided Wall Column). La cloison peut être fixe ou mobile, par exemple avec un garnissage structuré ou vrac.

Le flux (A) est de préférence à l'état liquide ou gazeux.

De préférence, le flux (A) comprend, voire est constitué de, méthylmercaptan, d'eau et éventuellement de traces de méthanol, d'H₂S et de sous-produits soufrés.

Le flux (A) peut comprendre au moins 90%, de préférence au moins 95%, plus préférentiellement au moins 98%, par exemple au moins 99% en poids de méthylmercaptan, par rapport au total en poids du méthylmercaptan et de l'eau.

Le flux (A) peut comprendre au moins 0,15% en poids d'eau, de préférence au moins strictement supérieur à 0,15% en poids d'eau, par rapport au poids total de l'eau et du méthylmercaptan. Le flux (A) peut comprendre au maximum 30%, de préférence au maximum 10% en poids d'eau, par rapport au total en poids du méthylmercaptan et de l'eau. Le flux (A) peut comprendre entre 0,15%, de préférence strictement supérieur à 0,15%, et 30% en poids d'eau par rapport au total en poids du méthylmercaptan et de l'eau.

Le flux (A) peut comprendre entre 0,15%, de préférence strictement supérieur à 0,15%, et 10% en poids d'eau par rapport au total en poids du méthylmercaptan et de l'eau.

De préférence, le flux (A) comprend entre 0,15%, de préférence strictement supérieur à 0,15%, et 5% en poids d'eau par rapport au total en poids du méthylmercaptan et de l'eau.

Par exemple, le flux (A) comprend entre 0,15%, de préférence strictement supérieur à 0,15% et 2%, par exemple entre 0,15% et 1,5% ou entre 0,15% et 1% en poids d'eau, par rapport au total en poids du méthylmercaptan et de l'eau ; le reste pouvant être du méthylmercaptan.

Suite à l'étape de distillation 2) du flux (A), on obtient un distillat (B) gazeux. Ce distillat (B) correspond notamment à un mélange azéotrope, de préférence hétéroazéotrope, en particulier dans les conditions de pression et/ou de température de l'étape de distillation 2).

Ainsi, la distillation de l'étape 2) permet notamment de former un mélange azéotrope (soit une distillation azéotropique). Une fois récupéré et condensé à l'état liquide (condensat (C)), il se retrouve sous forme biphasique, dont les deux phases peuvent être facilement séparées, notamment par décantation.

L'étape de condensation 4) du distillat (B) peut être réalisée par toute technique classique. La condensation peut être réalisée dans un condenseur séparé de la colonne de distillation ou qui peut être intégré à ladite colonne. On obtient alors un condensat (C) à l'état liquide, de préférence comprenant deux phases dont l'une est aqueuse et l'autre organique (et comprenant le méthylmercaptan). Lors de l'étape de condensation 4), la température peut être comprise entre 20°C et 50°C et/ou la pression peut être comprise entre 5 et 15 bars absolus.

Le distillat (B) et le condensat (C) ont de préférence la même composition.

Lors de l'étape 5) de séparation, toute méthode connue peut être utilisée. On utilise de façon tout à fait préférée la décantation. Lors de l'étape de séparation, la température peut être comprise entre 20°C et 50°C et/ou la pression peut être comprise entre 5 et 15 bars absolus. A l'issue de l'étape 5), on obtient deux phases liquides séparées :
- une phase aqueuse (D) ; et
- une phase organique (E) comprenant du méthylmercaptan.

Selon un mode de réalisation, la phase aqueuse (D) comprend :
- de l'eau,
- de l'H₂S, de préférence à l'état de traces,
- éventuellement du méthylmercaptan, de préférence à l'état de traces ; et
- éventuellement des sous-produits soufrés, de préférence à l'état de traces.

L'H₂S, et éventuellement le méthylmercaptan et les sous-produits soufrés, peuvent être séparés de ladite phase aqueuse. La séparation peut être réalisée par tout moyen connu et de préférence par un stripping, qui peut être un stripping thermique ou par gaz inerte (par exemple par entrainement à l'azote, au méthane, au CO₂). Cette phase gazeuse forme alors des évents appelés évents E3 ci-après.

Selon un mode de réalisation, les évents E3 sont incinérés et/ou la phase aqueuse (D) peut être éliminée vers le réseau des eaux usées.

Selon un autre mode de réalisation, les évents E3 peuvent être envoyés dans une colonne d'absorption au méthanol, afin de récupérer les composés soufrés tels que l'H₂S et/ou le méthylmercaptan, qu'ils comprennent par une extraction gaz(évents)-liquide(méthanol).

Selon un mode de réalisation, la phase organique (E) est récupérée à l'issue de l'étape 5) lorsque l'étape 6) de reflux n'est pas effectuée.

Selon un autre mode de réalisation, la phase organique (E) est utilisée en tout ou partie en tant que reflux de la colonne de distillation (1).

Lors de l'étape 6), le taux de reflux peut être compris entre 0 et 0,99, de préférence entre 0 et 0,60. On entend par « taux de reflux » le ratio massique [phase organique (E)/flux (A)].

Ainsi, le procédé selon l'invention permet d'obtenir un méthylmercaptan séché tel que défini ci-dessus.

Le procédé selon l'invention peut être réalisé en continu ou en batch, de préférence en continu.

Pendant les étapes 1) à 7) du procédé, la pression peut être comprise entre 0,05 et 75 bars absolus, de préférence entre 1 et 30 bars absolus, plus préférentiellement entre 5 et 15 bars absolus, par exemple à environ 10, 11, 12, 13, 14 ou 15 bars absolus.

Du méthanol, de préférence à l'état de traces, peut être compris dans le flux (A) et/ou le distillat (B) et/ou le condensat (C) et/ou la phase aqueuse (D) et/ou le flux (F).

Selon un mode de réalisation, le flux (A) est connecté à une unité de production de méthylmercaptan à partir de méthanol et d'hydrogène sulfuré.

Selon un mode de réalisation, le flux (A) est connecté à une unité de production de méthylmercaptan à partir d'au moins un oxyde de carbone, d'hydrogène et d'hydrogène sulfuré et/ou de soufre.

La présente invention concerne également l'utilisation d'une distillation azéotropique pour le séchage du méthylmercaptan. En particulier, ladite distillation azéotropique correspond à la distillation telle que décrite pour l'étape 2) du procédé de séchage selon l'invention.

La présente invention concerne également le méthylmercaptan séché tel que défini ci-dessus.

### Procédé de préparation de méthylmercaptan par voie méthanol

La présente invention concerne également un procédé de production de méthylmercaptan comprenant les étapes suivantes :
a) on fait réagir du méthanol avec de l'hydrogène sulfuré pour former un flux (M), de préférence à l'état gazeux, comprenant du méthylmercaptan, de l'eau, éventuellement de l'H₂S n'ayant pas réagi et des sous-produits soufrés ;
b) éventuellement, on condense ledit flux (M) ;
c) éventuellement on effectue au moins une étape de purification dudit flux (M) pour obtenir un flux enrichi en méthylmercaptan ; et
d) on effectue le séchage du flux obtenu à l'étape a), b) ou c) par le procédé de séchage tel que décrit ci-dessus.

De préférence, lors de l'étape c), ladite au moins une étape de purification correspond à au moins une étape de séparation de phases, de préférence par décantation, et/ou à au moins une étape de distillation.

L'étape c) peut notamment correspondre à une ou plusieurs étape(s) de séparation de phases, par exemple une ou deux étapes de décantation, et/ou une ou plusieurs étape(s) de distillation, par exemple une ou deux étapes de distillation.

En particulier, suite à l'étape c), on obtient un flux enrichi en méthylmercaptan et comprenant de l'eau.

De préférence, l'étape c) permet par une ou plusieurs étape(s) de purification d'éliminer du flux (M) l'H₂S, les sous-produits soufrés et la majeure partie de l'eau. De préférence au moins 50% en poids de l'eau, par exemple au moins 70%, voire au moins 90% en poids de l'eau est éliminée du flux (M) grâce à l'étape c). L'H₂S et les sous-produits soufrés peuvent rester à l'état de traces à l'issue de l'étape c).

Ledit procédé peut ainsi comprendre les étapes suivantes :
a) on fait réagir du méthanol avec de l'hydrogène sulfuré pour former un flux (M) comprenant du méthylmercaptan, de l'eau, de l'H₂S n'ayant pas réagi et des sous-produits soufrés ;
b) éventuellement, on condense ledit flux (M) ;
c1) on sépare, de préférence par décantation, à partir du flux (M) :
   - un flux gazeux (N) comprenant de l'hydrogène sulfuré n'ayant pas réagi ;
   - un flux aqueux (O) ; et
   - un flux (P) comprenant du méthylmercaptan, de l'eau, de l'hydrogène sulfuré n'ayant pas réagi et des sous-produits soufrés ;
c2) on effectue une distillation du flux (P) de façon à obtenir :
   - un flux (R) comprenant de l'hydrogène sulfuré, de préférence en tête de colonne ; et
   - un flux (S) comprenant du méthylmercaptan, de l'eau et des sous-produits soufrés, de préférence en fond de colonne ;
c3) on effectue une distillation du flux (S) de façon à obtenir :
   - un flux (T) comprenant du méthylmercaptan et de l'eau, de préférence en tête de colonne ; et
   - un flux (U) comprenant les sous-produits soufrés, de préférence en fond de colonne ;
c4) éventuellement, on sépare du flux (T) le méthylmercaptan de l'eau, de préférence par décantation, de façon à obtenir :
   - un flux (V) comprenant du méthylmercaptan et de l'eau ; et
   - un flux (W) comprenant de l'eau ;
d) on effectue le séchage du flux (T) ou (V) par le procédé de séchage tel que selon l'invention.

Ainsi, les étapes c1 à c4 sont des étapes de purification pour obtenir des flux de plus en plus riches en méthylmercaptan.

Lesdits flux (M) et/ou (P) et/ou (S) et/ou (T) et/ou (V) peuvent éventuellement comprendre du méthanol n'ayant pas réagi, de préférence à l'état de traces.

### Etape a) - Réaction :

Lors de l'étape a), on fait réagir le méthanol avec l'hydrogène sulfuré pour former un flux (M) comprenant du méthylmercaptan, de l'eau, éventuellement de l'H₂S n'ayant pas réagi et éventuellement des sous-produits soufrés.

Préalablement à l'étape a), on peut préparer un flux gazeux des réactifs H₂S et méthanol comme suit.

Du méthanol liquide est injecté dans de l'H₂S gazeux. Cette injection permet de vaporiser partiellement ou entièrement le méthanol. Le mélange d'H₂S et de méthanol peut ensuite être entièrement vaporisé si nécessaire de façon à obtenir un flux totalement gazeux.

Ainsi, un flux gazeux d'H₂S et de méthanol, de préférence préparé tel que ci-dessus, ou de façon séparée le méthanol et l'H₂S, chacun sous forme gazeuse, sont introduits dans un réacteur.

Ledit réacteur peut être isotherme ou adiabatique, à plaques, multitubulaire ou à lit fixe. On choisit de préférence un réacteur adiabatique.

La température de réaction peut être comprise entre 200° et 500°C, de préférence entre 200°C et 400°C. De préférence, la températurede réaction est comprise entre 200°C et 360°C. Au-dessus de cette température, le catalyseur peut être physiquement endommagé (par frittage et cokage notamment).

La pression peut être comprise entre 1 et 40 bars absolus.

Le rapport molaire H₂S/méthanol peut être compris entre 1 et 50, de préférence entre 1 et 25. L'H₂S est de préférence en excès par rapport au méthanol.

Le réacteur peut contenir un catalyseur pour la réaction de formation du méthylmercaptan, de préférence en phase gaz. Parmi les catalyseurs pouvant être utilisés, on peut citer :
- les catalyseurs à base d'alumine ;
- le dioxyde de thorium ThO₂, de préférence déposé sur un support silicaté ;
- les catalyseurs à base de sulfure de cadmium, de préférence sur un support alumine ;
- les catalyseurs à base des oxydes suivants : MgO, ZrO₂, TiO₂ rutile (R) et anatase (A), CeO₂, et γ-Al₂O₃ ;
- les catalyseurs à base d'oxydes de métaux, de préférence dopés par des métaux alcalins (Li, Na, K, Rb, Cs) et éventuellement supportés sur SiO₂, Al₂O₃ ou Nb₂O₅ ;
- les catalyseurs à base de carbonates de métaux alcalins ;
- les catalyseurs à base de sels de métaux alcalins avec certains acides de métaux de transitions (Cr, Mo, W, Ni), imprégnés sur l'alumine gamma ou autres oxydes de métaux ;
- le tungstate de potassium sur alumine K₂WO₄/Al₂O₃.

On obtient ainsi un flux (M) comprenant du méthylmercaptan, de l'eau, éventuellement de l'H₂S n'ayant pas réagi et des sous-produits soufrés.

### Etape b) - Condensation :

On peut éventuellement condenser le flux (M) issu de l'étape a) à l'aide toute technique classique, de préférence à l'aide d'un ou plusieurs condenseur(s) ou économiseur(s). Lors de la condensation, le flux (M) est notamment refroidi aussi bas que possible, pour maximiser l'élimination de l'eau, mais doit être maintenu strictement au-dessus de 16°C pour éviter la formation d'hydrates solides de méthylmercaptan. De préférence, le flux (M) est condensé à une température comprise entre 20°C et 70°C, par exemple entre 30°C et 60°C.

### Etape c) - Purification :

De préférence, lors de l'étape c), ladite au moins une étape de purification correspond à au moins une étape de séparation de phases, de préférence par décantation, et/ou à au moins une étape de distillation. L'étape c) peut notamment correspondre à une ou plusieurs étape(s) de séparation de phases, par exemple une ou deux étapes de décantation, et/ou une ou plusieurs étape(s) de distillation, par exemple une ou deux étapes de distillation.

De préférence, l'étape c) permet par une ou plusieurs étape(s) de purification d'éliminer du flux (M) l'H₂S n'ayant pas réagi et/ou les sous-produits soufrés et/ou l'eau. En particulier, suite à l'étape c), on obtient un flux enrichi en méthylmercaptan. L'étape de purification c) peut être effectuée par toute technique classique et en particulier selon les étapes c1) à c4) telles que décrites ci-dessous.

### Etape c1 - Séparation :

Lors de l'étape c1) de séparation, de préférence par décantation, on obtient :
- un flux gazeux (N) comprenant de l'hydrogène sulfuré n'ayant pas réagi ;
- un flux aqueux (O) ; et
- un flux (P) comprenant du méthylmercaptan, de l'eau, de l'hydrogène sulfuré n'ayant pas réagi et des sous-produits soufrés.

De préférence, le flux (M) est séparé à une température comprise entre 20°C et 70°C, de préférence entre 30°C et 60°C. La pression peutêtre comprise entre 1 et 40 bars absolus.

Le flux (P) obtenu peut notamment être à l'état gazeux ou à l'état liquide. Lorsque le flux (P) est à l'état gazeux, les flux (N) et (P) peuvent être combinés.

En particulier, le flux aqueux (O), de préférence à l'état liquide, comprend au moins 50%, de préférence au moins 70%, plus préférentiellement au moins 90% en poids d'eau, par rapport au poids total de l'eau présente dans le flux (M). Le flux aqueux (O) peut ensuite être envoyé vers un dégazeur. Le flux aqueux dégazé peut ensuite être envoyé au traitement des eaux usées.

Le flux gazeux (N) peut être recyclé à l'alimentation du réacteur de l'étape a). Dans ce cas, une purge de ce flux (N) est réalisée de façon à éviter l'accumulation d'inertes et/ou d'impuretés dans cette boucle de recyclage. On peut citer par exemple en tant qu'inertes et/ou impuretés : le méthane, CO, CO₂, H₂ et N₂. Le flux gazeux résultant de cette purge est appelé Events E1. Lorsque les flux (N) et (P) sont combinés, le même type de purge peut être effectuée de façon à obtenir un flux gazeux appelé Events E1'.

Selon un mode de réalisation, les Events E1 ou E1' sont envoyés à l'incinération.

Selon un autre mode de réalisation, les évents E1 ou E1' peuvent être envoyés dans une colonne d'absorption au méthanol, afin de récupérer les composés soufrés tels que l'H₂S et/ou le méthylmercaptan, qu'ils comprennent par une extraction gaz(évents)-liquide(méthanol).

### Etape c2 - Elimination de l'H₂S par distillation

On effectue ensuite une distillation du flux (P) de façon à obtenir :
- un flux (R) comprenant de l'hydrogène sulfuré, de préférence en tête de colonne ; et
- un flux (S) comprenant du méthylmercaptan, de l'eau et des sous-produits soufrés, de préférence en fond de colonne ;

Lors de la distillation, la pression peut être comprise entre 1 et 40 bar absolus et/ou la température peut être comprise entre -60°C et +60°C en tête de colonne, et entre +20°C et +200°C en fond de colonne.

Le flux (R) comprenant l'H₂S peut être récupéré en tête de colonne, et éventuellement recyclé vers l'alimentation du réacteur de l'étape a).

En particulier, ladite distillation de l'étape c2) permet d'éliminer l'H₂S restant dans le flux (P) (il est entendu que des traces d'H₂S peuvent être rester dans le flux (S)).

### Etape c3 - Elimination des sous-produits soufrés par distillation :

On effectue une distillation du flux (S) de façon à obtenir :
- un flux (T) comprenant du méthylmercaptan et de l'eau, de préférence en tête de colonne ; et
- un flux (U) comprenant les sous-produits soufrés, de préférence en fond de colonne.

Lors de la distillation, la pression peut être comprise entre 1 et 40 bar absolus et/ou la température peut être comprise entre +20°C et +100°C en tête de colonne, et entre +40°C et +200°C en fond de colonne.

En particulier, ladite distillation de l'étape c3) permet d'éliminer les sous-produits soufrés restant dans le flux (S) (il est entendu que des traces des sous-produits soufrés peuvent rester dans le flux (T)).

### Etape c4 - Séparation du méthylmercaptan et de l'eau :

Préalablement à l'étape c4), le flux (T) peut être refroidi aussi bas que possible, pour maximiser l'élimination de l'eau, mais doit être maintenu strictement au-dessus de 16°C pour éviter la formation d'hydrates solides deméthylmercaptan. De préférence, le flux (T) est refroidi à une température comprise entre 20°C et 70°C, par exemple entre 30°C et 60°C.

Ce refroidissement permet de maximiser la séparation de l'eau lors de l'étape c4), tout en maintenant une température strictement supérieure à 16°C pour éviter la formation d'hydrates solides de méthylmercaptan.

On peut ensuite effectuer une séparation du méthylmercaptan et de l'eau restante, de préférence par décantation, de façon à obtenir :
- un flux (V) comprenant du méthylmercaptan et de l'eau, de préférence à l'état liquide ;
- un flux (W) comprenant de l'eau, de préférence à l'état liquide.

En particulier, lors de l'étape c4), le flux (W) comprend au moins 50% en poids, de préférence au moins 70%, plus préférentiellement au moins 90% en poids d'eau, par rapport au poids total de l'eau présente dans le flux (T).

Le flux (T) ou le flux (V) correspond notamment au flux (A) tel que défini ci-dessus. Le flux (V) ou le flux (T) obtenu peut ensuite être séché selon le procédé de séchage tel que selon l'invention.

Lors de l'étape de séparation c4), il est possible de récupérer la phase gazeuse ainsi séparée des flux (W) et (V), qui sont tous deux à l'état liquide. Ce flux gazeux est appelé Events E2.

Selon un mode de réalisation, les évents E2 sont incinérés.

Selon un autre mode de réalisation, les évents E2 peuvent être envoyés dans une colonne d'absorption au méthanol, afin de récupérer les composés soufrés tels que l'H₂S et/ou le méthylmercaptan, qu'ils comprennent par une extraction gaz(évents)-liquide(méthanol).

### Procédé de préparation de méthylmercaptan par voie oxyde(s) de carbone

Le procédé de production de méthylmercaptan par voie oxyde(s) de carbone est réalisé à partir d'au moins un oxyde de carbone, d'hydrogène et d'hydrogène sulfuré et/ou de soufre. L'oxyde de carbone est choisi parmi le monoxyde de carbone (CO) et le dioxyde de carbone (CO₂). De préférence, l'oxyde de carbone est le monoxyde de carbone (CO).

Ledit procédé est ainsi préférentiellement réalisé à partir d'un mélange de monoxyde de carbone, d'hydrogène et d'hydrogène sulfuré. Le sous-produit principal de cette synthèse est le dioxyde de carbone (CO₂).

L'oxysulfure de carbone (COS) est considéré comme l'intermédiaire réactionnel qui mène au méthylmercaptan après hydrogénation selon les réactions suivantes :

CO + H₂S -> COS + H₂

COS + 3 H₂ -> CH₃SH + H₂O

Le CO₂ est quant à lui issu de plusieurs réactions secondaires telles que :

CO + H₂O -> CO₂ + H₂

COS + H₂O -> CO₂ + H₂S

2 COS -> CO₂ + CS₂

Le dioxyde de carbone obtenu peut éventuellement être recyclé pour produire également du méthylmercaptan, selon l'équation suivante :

CO₂ + 3 H₂ + H₂S -> CH₃SH + 2 H₂O

Un tel procédé de production de méthylmercaptan est largement décrit, comme par exemple dans les demandes EP 0171312 ou WO 08/125452.

La présente invention concerne ainsi un procédé de production de méthylmercaptan comprenant les étapes suivantes :
a-ox) on fait réagir, de préférence à l'état gazeux, au moins un oxyde de carbone, de l'H₂, de l'H₂S et/ou du soufre en présence d'au moins un catalyseur afin de former un flux (J), de préférence à l'état gazeux, comprenant du méthylmercaptan, de l'eau et éventuellement ledit au moins un oxyde de carbone, de l'H₂, de l'H₂S n'ayant pas réagi et du sulfure de carbonyle (COS) ;
b-ox) éventuellement on condense ledit flux (J) ;
c-ox) éventuellement on effectue au moins une étape de purification dudit flux (J) pour obtenir un flux enrichi en méthylmercaptan ; et
d-ox) on effectue le séchage du flux obtenu à l'étape a-ox), b-ox) ou c-ox) par le procédé de séchage tel que défini ci-dessus.

Lorsque l'oxyde de carbone est le CO, le flux (J) peut comprendre du CO n'ayant pas réagi et du CO₂ formé lors de l'étape a-ox).

En particulier, ledit procédé de production de méthylmercaptan comprend les étapes suivantes :
a-ox) on fait réagir, de préférence à l'état gazeux, au moins un oxyde de carbone, de l'H₂, de l'H₂S et/ou du soufre en présence d'au moins un catalyseur afin de former un flux (J), de préférence à l'état gazeux, comprenant du méthylmercaptan, de l'eau et ledit au moins un oxyde de carbone, de l'H₂, de l'H₂S n'ayant pas réagi et du sulfure de carbonyle (COS) ;
b-ox) on condense ledit flux (J) ;
c1-ox) on sépare, de préférence par décantation, à partir du flux (J) liquide :
   - une phase organique (K) liquide comprenant le méthylmercaptan et de l'eau ; et
   - une phase aqueuse (L) liquide ;
c2-ox) éventuellement, on effectue une séparation des composés incondensables, de façon à obtenir un flux (J'), de préférence à l'état gazeux ; ladite séparation pouvant être réalisée simultanément à l'étape b-ox ou à l'étape c1-ox) ;
d-ox) on effectue le séchage du flux (K) selon le procédé de séchage tel que défini ci-dessus ; et
e-ox) éventuellement on recycle le flux (J') à l'étape a-ox).

Ainsi, les étapes c1-ox et c2-ox sont notamment des étapes de purification pour obtenir des flux de plus en plus riches en méthylmercaptan.

Selon un mode de réalisation, le flux (J) ou le flux (K) correspond au flux (A) tel que selon l'invention.

### Etape a-ox) - Réaction :

L'étape de réaction a) est bien connue. En particulier, l'étape a-ox) est effectuée à une température comprise entre 200°C et 500°C, de péférence entre 200°C et 400°C. En particulier, l'étape a-ox) est effectuée à une pression comprise entre 1 et 100 bars absolus, de préférence entre 3 et 30 bars absolus.

De préférence, lors de l'étape a-ox), le ratio molaire oxyde de carbone/S/H₂S/H₂ est compris entre 1/0/0,05/0,05 et 1/20/40/100, De préférence il est entre 1/0/0,5/1 et 1/0/10/20. En particulier, il est de 1/0/1/2.

De préférence, lors de l'étape a-ox) en l'absence de soufre, le ratio CO/H₂/H₂S est compris 1/0,05/0,05 et 1/40/100, De préférence il est entre 1/0,5/1 et 1/10/20. En particulier, il est de 1/2/1.

L'étape a-ox) peut être réalisée sur un ou plusieurs lit(s) catalytique(s), de préférence fixe(s). Elle peut être réalisée dans un réacteur comprenant une ou plusieurs zones réactionnelles, le(s) réactif(s) pouvant être alimenté(s) entre les différentes zones. Ainsi, les réactifs, de préférence l'H₂ et/ou l'H₂S, peuvent être introduits séparément sur les différents lits catalytiques ou zones réactionnelles.

Ledit au moins un catalyseur utilisé à l'étape a-ox) est connu et peut notamment être choisi parmi :
- les catalyseur à base de molybdène et de potassium supportés sur zircone tels que K₂MoO₄/ZrO₂, comme décrits dans le document WO 2019/122072. Ces catalyseurs sont testés à une température de 320 °C et à une pression de 10 bars en utilisant un ratio CO/H₂/H₂S de 1/2/1.
- les catalyseurs à base de molybdène et de potassium de type Mo-S-K et/ou Mo-O-K sur un support d'hydroxyapatite tels que K₂MoS₄/Ca₁₀(PO₄)₆(OH)₂ ou K₂MoO₄/Ca₁₀(PO₄)₆(OH)₂ comme décrits dans le document WO 2014/154885. Ces catalyseurs sont testés à une température de 280 °C et à une pression de 10 bars en utilisant un ratio CO/H₂/H₂S de 1/2/1.
- Les catalyseurs décrits dans la demande de brevet US 2010/0286448 composés d'un support poreux tel que SiO₂, TiO₂, les silico-alumines, les zéolithes et les nanotubes de carbone, sur lequel a été déposé électrolytiquement un métal. K₂MoO₄, ainsi qu'un autre oxyde métallique jouant le rôle de promoteur, sont ensuite imprégnés sur ce support.
- les catalyseurs à base de Mo et de K (en particulier K₂MoO₄) promus par TeO₂ et supportés tels que K₂MoO₄/TeO₂/SiO₂, décrits dans le document US 2010/094059.
   Le catalyseur K₂MoO₄/TeO₂/SiO₂ est testé pour une température de 300°C et à une pression de 2 bars, en prenant un ratio CO/H₂/H₂S de 1/1/2 et une vitesse spatiale horaire de 2000 h-1.
- La demande internationale WO 2005/040082 décrit plusieurs catalyseurs et notamment un catalyseur comprenant un composant actif à base de Mo-O-K, un promoteur actif et éventuellement un support. Les catalyseurs exemplifiés sont K₂MoO₄/Fe₂O₃/NiO ou encore K₂MoO₄/CoO/CeO₂/SiO₂, chacun supporté sur de la silice. Ces catalyseurs sont testés à une température de 320 °C et à une pression de 7 bars, en prenant un ratio CO/H₂/H₂S de 1/1/2 et une vitesse spatiale horaire de 3000 h⁻¹.

### Etape b-ox) - Condensation :

Tout type de condenseur peut être utilisé pour cette opération tels que les échangeurs à plaques ou tubulaires. De préférence, le condenseur est à fluides séparés, c'est à dire sans contact entre les gaz à condenser et le fluide réfrigérant. Le fluide réfrigérant peut être liquide ou gazeux tels que l'air, l'eau, la saumure, l'ammoniac, des fréons, des huiles...

La température de condensation peut être comprise entre 20°C et 70°C, de préférence entre 30°C et 60°C. La pression peut ête comprise entre 1 bar absolu et 100 bars absolus. L'objectif est de condenser un maximum de méthylmercaptan et d'eau par rapport aux composés incondensables (tels que CO/COS/CO₂/H₂/H₂S), ce qui permettra une séparation des phases liquide et gazeuse facile.

### Etape c-ox) - Purification :

### Etape c1-ox) - Séparation de l'eau

L'étape de séparation c1-ox) peut être effectuée par toute technique classique et en particulier par décantation. De préférence, le flux (J) est à l'état liquide. Ainsi, on sépare, de préférence par décantation, à partir du flux (J) :
- une phase organique (K) comprenant le méthylmercaptan et de l'eau ; et
- une phase aqueuse (L).

En particulier, lors de l'étape c1-ox), la phase aqueuse (L) comprend au moins 50% en poids, de préférence au moins 70%, plus préférentiellement au moins 90% en poids d'eau, par rapport au poids total de l'eau présente dans le flux (J).

### Etape c2-ox - Séparation des incondensables :

On entend notamment par composés incondensables, les composés restant à l'état gazeux aux températures et pressions dudit procédé de production, notamment après l'étape de condensation b-ox). On peut notamment citer comme incondensable l'oxyde de carbone (CO et/ou CO₂), l'H₂, l'H₂S, le sulfure de carbonyle (COS), le méthane et tout autre composé inerte incondensable produit ou introduit lors dudit procédé.

La séparation peut être effectuée par toute technique classique. On obtient notamment un flux (J') à l'état gazeux comprenant les composés incondensables tels que l'oxyde de carbone (CO et/ou CO₂), l'H₂, l'H₂S, le sulfure de carbonyle (COS), le méthane et tout autre composé inerte incondensable produit ou introduit lors dudit procédé.

Selon un mode de réalisation, on peut recycler le flux (J') à l'étape a-ox), de préférence directement (sans étape de purification intermédiaire). Selon un autre mode de réalisation, le flux (J') peut être en partie purgé. S'il n'est pas recyclé, il peut être envoyé à l'incinérateur ou toute autre unité de traitement de gaz.

En particulier, que le procédé de production de méthylmercaptan soit par voie méthanol ou par voie oxyde(s) de carbone, ces derniers peuvent chacun comprendre au moins une étape de purification telle que définie ci-dessus qui est une étape de séparation de l'eau et du méthylmercaptan préalable au séchage, et de préférence par décantation (par exemple l'étape c1 et/ou c4 et c1-ox respectivement).

En particulier, une telle étape permet de séparer l'eau du méthylmercaptan de façon à obtenir un méthylmercaptan avec une teneur en eau résiduelle, c'est-à-dire d'obtenir un méthylmercaptan avec teneur en eau qui dépend de la solubilité de l'eau dans le méthylmercaptan, à la température de séparation. Généralement, cette teneur est comprise entre 0,15%, de préférence strictement supérieur à 0,15%, et 30% en poids d'eau par rapport au total en poids du méthylmercaptan et de l'eau, par exemple entre 0,15% et 10%. De préférence, elle est comprise entre 0,15%, de préférence strictement supérieur à 0,15%, et 5% en poids d'eau par rapport au total en poids du méthylmercaptan et de l'eau. Par exemple, elle est comprise entre 0,15%, de préférence strictement supérieur à 0,15% et 2%, par exemple entre 0,15% et 1,5% ou entre 0,15% et 1% d'eau, par rapport au total en poids du méthylmercaptan et de l'eau.

Suite à cette étape, le procédé de séchage du méthylmercaptan tel que décrit ci-dessus peut être mis en œuvre de façon plus efficace et économique, la quantité d'eau avant séchage ayant été réduite au maximum.

### Description des Figures

### Figure 1 :

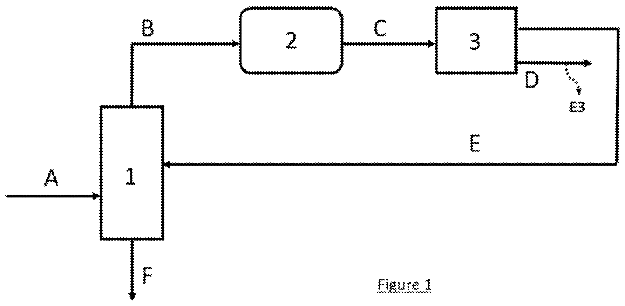

La Figure 1 représente un mode de réalisation du procédé de séchage selon l'invention.

Le flux (A) entre dans la colonne de distillation (1). Le flux (A) est distillé dans la colonne (1). Le distillat (B) est récupéré en tête de colonne sous forme gazeuse. Le distillat (B) est ensuite condensé dans un condenseur (2) où il est récupéré sous forme liquide biphasique (condensat (C)). Le condensat (C) décante ensuite dans le décanteur (3) afin d'obtenir :
- une phase aqueuse (D), et
- une phase organique (E).

La phase organique (E) sert ensuite de reflux à la colonne de distillation (1).

Le méthylmercaptan séché est récupéré en fond de colonne (1) (flux (F)).

### Figure 2 :

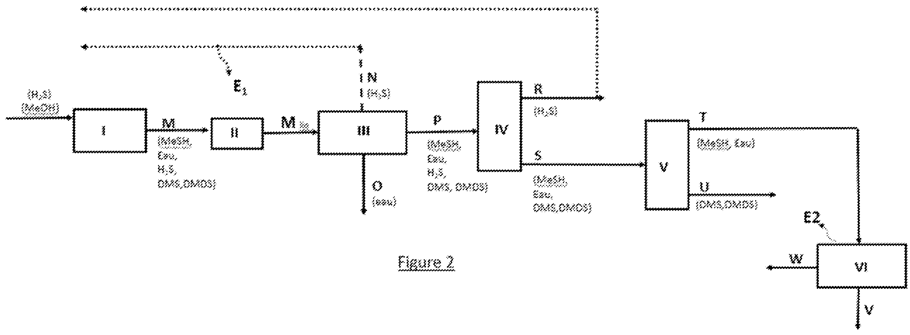

La Figure 2 représente un mode de réalisation d'un procédé de production de méthylmercaptan par voie méthanol.

L'étape a) de réaction est réalisée dans un réacteur (I) à partir de méthanol et d'H₂S. Le flux (M) sortant du réacteur (I) comprend du MeSH, de l'eau, de l'H₂S et des sous-produits soufrés. Le flux (M) est condensé dans un condenseur (II). Il est ensuite séparé dans un décanteur (III) en trois flux :
- un flux (N) comprenant de l'H₂S,
- un flux (O) comprenant de l'eau, et
- un flux (P) comprenant du MeSH, de l'eau, de l'H₂S et des sous-produits soufrés. Le flux (P) est distillé dans une colonne de distillation (IV) pour éliminer l'H₂S (flux (R) en tête de colonne) et obtenir un flux (S) en pied de colonne comprenant du MeSH, de l'eau et des sous-produits soufrés. Le flux (S) est ensuite distillé dans une colonne de distillation (V) pour obtenir un flux (U) en pied de colonne comprenant les sous-produits soufrés et un flux (T) en tête de colonne comprenant le MeSH et l'eau. Le flux (T) est ensuite séparé dans un décanteur (VI) en un flux (V) comprenant le MeSH et l'eau et un flux (W) comprenant de l'eau.

### Figure 3 :

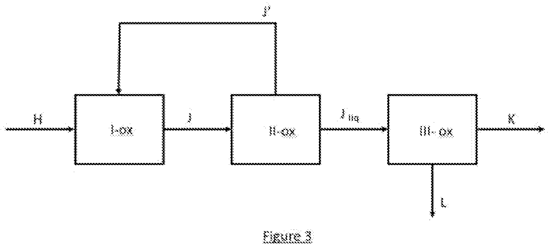

La Figure 3 représente un mode de réalisation d'un procédé de production de méthylmercaptan par voie oxyde de carbone.

Un flux (H) comprenant du CO, de l'hydrogène et de l'H₂S est introduit à l'état gazeux dans un réacteur I-ox de façon à récupérer en sortie un flux (J) comprenant du méthylmercaptan, de l'eau et éventuellement du CO, du CO₂, de l'H₂, de l'H₂S n'ayant pas réagi et du sulfure de carbonyle (COS).

Le flux (J) est condensé puis séparé dans le condenseur II-ox de façon à obtenir un flux (J) à l'état liquide enrichi en méthylmercaptan et un flux (J') à l'état gazeux comprenant du CO, du CO₂, de l'H₂, de l'H₂S n'ayant pas réagi et du sulfure de carbonyle (COS) ;

Le flux (J') est ensuite recyclé dans le réacteur I-ox. On sépare ensuite dans un décanteur III-ox, à partir du flux (J) :
- une phase organique (K) comprenant le méthylmercaptan et de l'eau ; et
- une phase aqueuse (L).

Le séchage du flux (K) est ensuite effectué selon le procédé de séchage de l'invention.

Les exemples qui suivent permettent d'illustrer la présente invention mais ne sont en aucun cas limitatifs.

### EXEMPLES

### Exemple 1 : Essai comparatif avec les tamis moléculaires

Le séchage en continu sur tamis moléculaire nécessite au minimum 2 sécheurs en parallèle (le 2^{ème} en régénération quand le premier est en adsorption).

On utilise une colonne d'adsorption (sécheur) qui contient 1 kg de tamis moléculaire Siliporite RA (1/8 de pouce en granulométrie), le débit de méthylmercaptan à sécher est de 1 kg/h. Les compositions entrée et sortie de ce sécheur sont les suivantes :

**[Table 1]**

| | Entrée | Sortie |
|---|---|---|
| Dimethylsulfure (DMS - ppm) Eau (ppm) | **153** | **550** |
| Methylcarptan | 3871 | 80 |
| (MeSH - %) Méthanol (MeOH - ppm) | 99.38 | 99.89 |
| | 1962 | 176 |
| TOTAL (%) | 99.98 | 99.97 |

Lorsque des tamis moléculaires sont utilisés, on peut observer que la quantité de diméthylsulfure (DMS) a été multipliée par 3 après séchage et que la quantité de méthanol a été divisée par presque 10. En effet, le méthanol s'adsorbe sur les tamis moléculaires avec pour conséquence de réduire considérablement la capacité de séchage en eau de ces tamis, et de ce fait d'augmenter la fréquence des cycles adsorption/régénération.

### Exemple 2 : Procédé de séchage selon l'invention

Le procédé de séchage correspond à celui décrit pour la Figure 1.

On fait entrer dans une colonne de distillation un flux de MeSH à sécher comprenant 99,77% en poids de MeSH (1000 kg/h) et 0,23% en poids d'eau (2,3 kg/h), par rapport au poids total du MeSH et de l'eau.

La colonne de distillation azéotropique comprend 28 plateaux et répond aux critères suivants :
- la pression de distillation est de 13 bars absolus ;
- le profil de température est entre 90 °C en bas et 70 °C en tête de colonne ;
- le taux de reflux est à 47%.

Le distillat est récupéré en tête de colonne sous forme gazeuse. Il comprend 98,88% en poids de MeSH (467,4 kg/h) et 1,12% en poids d'eau (5,3 kg/h), par rapport au poids total MeSH et eau (472,7kg/h). La température du distillat est d'environ 72°C pour une pression d'environ 12 bars absolus.

Le distillat est ensuite condensé dans un condenseur. Sa composition reste identique et il est récupéré sous forme liquide biphasique à une température d'environ 40°C pour une pression d'environ 12 bars absolus. Le condensat décante ensuite dans un décanteur afin d'obtenir :
- une phase aqueuse comprenant 98,26% (2,26kg/h) en poids d'eau et 1,74% en poids de MeSH (0,04kg/h) par rapport au poids total de la phase aqueuse (2,3 kg/h), et
- une phase organique comprenant 99,36% en poids de MeSH (467,5 kg/h) et 0,64% en poids d'eau (3 kg/h), par rapport au poids total MeSH et eau (470,5 kg/h). La température est d'environ 40°C pour une pression d'environ 12 bars absolus pour les deux phases.

Le MeSH seché est récupéré en de fond de la colonne de distillation et contient moins de 10 ppm en poids d'eau par rapport au total en poids du méthylmercaptan et de l'eau.

Les quantités de méthanol et de diméthylsulfure sont les mêmes à l'entrée du flux (A) que dans le méthylmercaptan séché (environ 0,04 kg/h et 0,1 kg/h respectivement).

Par conséquent, le procédé selon l'invention permet de sécher efficacement le méthylmercaptan tout en n'augmentant pas la quantité du sous-produit DMS. De plus, le procédé de séchage n'est pas affecté par les traces de méthanol et peut être réalisé en continu sans voir ses performances altérées.

## Revendications

1. Procédé de séchage de méthylmercaptan comprenant les étapes suivantes :
1) on introduit un flux (**A**) comprenant du méthylmercaptan et de l'eau dans une colonne de distillation (**1**) ;
2) on distille ledit flux (**A**) dans ladite colonne (**1**) ;
3) on récupère le distillat (**B**) à l'état gazeux, de préférence en tête de colonne ;
4) on condense le distillat (**B**), de préférence dans un condenseur (**2**), de façon à obtenir un condensat (**C**) à l'état liquide ;
5) on sépare, de préférence à l'aide d'un décanteur (**3**), ledit condensat (**C**) de façon à obtenir deux phases liquides séparées :
- une phase aqueuse (**D**) ; et
- une phase organique (**E**) comprenant du méthylmercaptan ;
6) éventuellement on introduit en tout ou partie la phase organique (**E**) dans la colonne de distillation (1) en tant que reflux ; et
7) on récupère un flux (**F**) comprenant le méthylmercaptan séché en fond de colonne (**1**).

2. Procédé de séchage selon la revendication 1, dans lequel la distillation de l'étape 2) est effectuée à une pression comprise entre 0,05 et 75 bars absolus, de préférence entre 1 et 30 bars absolus, plus préférentiellement entre 5 et 15 bars absolus.

3. Procédé de séchage selon la revendication 1 ou 2, dans lequel la distillation de l'étape 2) est effectuée à une température comprise entre 20°C et 200°C, de préférence entre 60°C et 100°C, plus préférentiellement entre 65°C et 95°C.

4. Procédé de séchage selon l'une quelconque des revendications précédentes, dans lequel la distillation de l'étape 2) est une distillation azéotropique.

5. Procédé de séchage selon l'une quelconque des revendications précédentes, dans lequel le flux (A) comprend au moins 90%, de préférence au moins 95%, plus préférentiellement au moins 98%, par exemple au moins 98,5%, voire au moins 99% en poids de méthylmercaptan, par rapport au total en poids du méthylmercaptan et de l'eau.

6. Procédé de séchage selon l'une quelconque des revendications précédentes, dans lequel la quantité d'eau dans le flux (F) est comprise entre 0 et 1500 ppm, de préférence est comprise entre 0 et 1000 ppm, plus préférentiellement entre 40 et 800 ppm, par rapport au total en poids du méthylmercaptan et de l'eau.

7. Procédé de séchage selon l'une quelconque des revendications précédentes, dans lequel lorsque l'étape 6) est effectuée, le taux de reflux est compris entre 0 et 0,99, de préférence entre 0 et 0,60.

8. Procédé de séchage selon l'une quelconque des revendications précédentes, dans lequel le flux (A) est connecté à une unité de production de méthylmercaptan à partir de méthanol et d'hydrogène sulfuré.

9. Procédé de séchage selon l'une quelconque des revendications précédentes, dans lequel le flux (A) est connecté à une unité de production de méthylmercaptan à partir d'oxyde de carbone, d'hydrogène (H₂), d'hydrogène sulfuré (H₂S) et/ou de soufre (S).

10. Procédé de production de méthylmercaptan comprenant les étapes suivantes :
a) on fait réagir du méthanol avec de l'hydrogène sulfuré pour former un flux (M), de préférence à l'état gazeux, comprenant du méthylmercaptan, de l'eau et éventuellement de l'H₂S n'ayant pas réagi et des sous-produits soufrés ;
b) éventuellement, on condense ledit flux (M) ;
c) on effectue au moins une étape de purification dudit flux (M) pour obtenir un flux enrichi en méthylmercaptan ;
d) on effectue le séchage du flux obtenu à l'étape c) par le procédé de séchage selon l'une quelconque des revendications 1 à 7.

11. Utilisation d'une distillation azéotropique pour le séchage du méthylmercaptan.

## Patentansprüche

1. Verfahren zum Trocknen von Methylmercaptan, umfassend die folgenden Schritte:
1) Einleiten eines Stroms (A), der Methylmercaptan und Wasser umfasst, in eine Destillationskolonne (1);
2) Destillieren des Stroms (A) in der Kolonne (1);
3) Rückgewinnen des Destillats (B) in gasförmigem Zustand, vorzugsweise am Kolonnenkopf;
4) Kondensieren des Destillats (B), vorzugsweise in einem Kondensator (2), so dass ein Kondensat (C) in flüssigem Zustand erhalten wird;
5) Trennen des Kondensats (C), vorzugsweise mithilfe eines Dekanters (3), so dass zwei getrennte flüssige Phasen erhalten werden:
- eine wässrige Phase (D); und
- eine organische Phase (E), die Methylmercaptan umfasst;
6) gegebenenfalls Einleiten der gesamten oder eines Teils der organischen Phase (E) als Rücklauf in die Destillationskolonne (1); und
7) Rückgewinnen eines Stroms (F), der das getrocknete Methylmercaptan umfasst, am Kolonnensumpf (1).

2. Verfahren zum Trocknen nach Anspruch 1, wobei die Destillation des Schritts 2) bei einem Druck zwischen 0,05 und 75 bar absolut, vorzugsweise zwischen 1 und 30 bar absolut, besonders bevorzugt zwischen 5 und 15 bar absolut, durchgeführt wird.

3. Verfahren zum Trocknen nach Anspruch 1 oder 2, wobei die Destillation des Schritts 2) bei einer Temperatur zwischen 20 °C und 200 °C, vorzugsweise zwischen 60 °C und 100 °C, besonders bevorzugt zwischen 65 °C und 95 °C, durchgeführt wird.

4. Verfahren zum Trocknen nach einem der vorhergehenden Ansprüche, wobei die Destillation des Schritts 2) eine azeotrope Destillation ist.

5. Verfahren zum Trocknen nach einem der vorhergehenden Ansprüche, wobei der Strom (A) mindestens 90 %, vorzugsweise mindestens 95 %, besonders bevorzugt mindestens 98 %, zum Beispiel mindestens 98,5 % oder sogar mindestens 99 Gew.-% Methylmercaptan bezogen auf das Gesamtgewicht des Methylmercaptans und des Wassers umfasst.

6. Verfahren zum Trocknen nach einem der vorhergehenden Ansprüche, wobei die Wassermenge in dem Strom (F) zwischen 0 und 1500 ppm, vorzugsweise zwischen 0 und 1000 ppm, besonders bevorzugt zwischen 40 und 800 ppm, bezogen auf das Gesamtgewicht des Methylmercaptans und des Wassers beträgt.

7. Verfahren zum Trocknen nach einem der vorhergehenden Ansprüche, wobei, wenn der Schritt 6) durchgeführt wird, das Rücklaufverhältnis zwischen 0 und 0,99, vorzugsweise zwischen 0 und 0,60, liegt.

8. Verfahren zum Trocknen nach einem der vorhergehenden Ansprüche, wobei der Strom (A) an eine Einheit zur Produktion von Methylmercaptan aus Methanol und Schwefelwasserstoff angeschlossen ist.

9. Verfahren zum Trocknen nach einem der vorhergehenden Ansprüche, wobei der Strom (A) an eine Einheit zur Produktion von Methylmercaptan aus Kohlendioxid, Wasserstoff (H₂), Schwefelwasserstoff (H₂S) und/oder Schwefel (S) angeschlossen ist.

10. Verfahren zur Produktion von Methylmercaptan, umfassend die folgenden Schritte:
a) Umsetzen von Methanol mit Schwefelwasserstoff, um einen Strom (M), vorzugsweise in gasförmigem Zustand, zu bilden, der Methylmercaptan, Wasser und gegebenenfalls nicht umgesetztes H₂S und schwefelhaltige Nebenprodukte umfasst;
b) Gegebenenfalls Kondensieren des Stroms (M);
c) Durchführen mindestens eines Schritts zum Aufreinigen des Stroms (M), um einen an Methylmercaptan angereicherten Strom zu erhalten;
d) Durchführen des Trocknens des im Schritt c) erhaltenen Stroms durch das Verfahren zum Trocknen nach einem der Ansprüche 1 bis 7.

11. Verwendung einer azeotropen Destillation zum Trocknen von Methylmercaptan.

## Claims

1. Process for drying methyl mercaptan, comprising the following steps:
1) a stream (A) comprising methyl mercaptan and water is introduced into a distillation column (1);
2) said stream (A) is distilled in said column (1);
3) the distillate (B) is recovered in gaseous form, preferably at the top of the column;
4) the distillate (B) is condensed, preferably in a condenser (2), so as to obtain a condensate (C) in liquid form;
5) said condensate (C) is separated, preferably using a decanter (3), so as to obtain two separate liquid phases:
- an aqueous phase (D); and
- an organic phase (E) comprising methyl mercaptan;
6) all or part of the organic phase (E) is optionally introduced into the distillation column (1) as reflux; and
7) a stream (F) comprising the dried methyl mercaptan is recovered at the bottom of column (1).

2. Drying process according to Claim 1, in which the distillation of step 2) is performed at a pressure of between 0.05 and 75 bar absolute, preferably between 1 and 30 bar absolute, more preferentially between 5 and 15 bar absolute.

3. Drying process according to Claim 1 or 2, in which the distillation of step 2) is performed at a temperature of between 20°C and 200°C, preferably between 60°C and 100°C, more preferentially between 65°C and 95°C.

4. Drying process according to any one of the preceding claims, in which the distillation of step 2) is an azeotropic distillation.

5. Drying process according to any one of the preceding claims, in which stream (A) comprises at least 90%, preferably at least 95%, more preferentially at least 98%, for example at least 98.5%, or even at least 99% by weight, of methyl mercaptan, relative to the total by weight of methyl mercaptan and water.

6. Drying process according to any one of the preceding claims, in which the amount of water in stream (F) is between 0 and 1500 ppm, preferably is between between 0 and 1000 ppm, more preferentially between 40 and 800 ppm, relative to the total by weight of methyl mercaptan and water.

7. Drying process according to any one of the preceding claims, in which, when step 6) is performed, the reflux ratio is between 0 and 0.99, preferably between 0 and 0.60.

8. Drying process according to any one of the preceding claims, in which stream (A) is connected to a unit for producing methyl mercaptan from methanol and hydrogen sulfide.

9. Drying process according to any one of the preceding claims, in which stream (A) is connected to a unit for producing methyl mercaptan from carbon oxide, hydrogen (H₂), hydrogen sulfide (H₂S) and/or sulfur (S).

10. Process for producing methyl mercaptan, comprising the following steps:
a) reacting methanol with hydrogen sulfide to form a stream (M), preferably in gaseous form, comprising methyl mercaptan, water, and possibly unreacted H₂S and sulfur byproducts;
b) optionally, said stream (M) is condensed;
c) at least one step of purification of said stream (M) is performed to obtain a stream enriched in methyl mercaptan;
d) the stream obtained in step c) is dried via the drying process according to any one of Claims 1 to 7.

11. Use of azeotropic distillation for drying methyl mercaptan.
